# EUROPÄISCHE PATENTSCHRIFT

(11) **EP 2 110 980 B1**
(45) Veröffentlichungstag und Bekanntmachung des Hinweises auf die Patenterteilung: **14.10.2020**
(21) Anmeldenummer: 09005545.0
(22) Anmeldetag: 20.04.2009
(51) Int. Cl.: H04L 9/00, H04L 9/08

(54) **Sichere serverbasierte Speicherung und Freigabe von Daten**
Secure server-based storage and release of data
Stockage sûr basé sur un serveur et validation de données

(30) Priorität: 18.04.2008 DE 102008019627
(43) Veröffentlichungstag der Anmeldung: 21.10.2009
(73) Patentinhaber: Samedi GmbH, 10115 Berlin (DE)
(72) Erfinder: Keller, Katrin, 10119 Berlin (DE); Alscher, Alexander, Dr., 10119 Berlin (DE); Alexander, Levin, 13125 Berlin (DE)
(74) Vertreter: Lang, Johannes

(56) Entgegenhaltungen:
- US-A- 1 010 689
- US-A1- 2002 076 042
- US-B1- 6 694 025
- US-B1- 6 834 112
- US-B1- 7 010 689
- MAHESH KALLAHALLA* ERIK RIEDELT RAM SWAMINATHAN* QIAN WANGT KEVIN FU PRG: "Plutus: Scalable secure file sharing on untrusted storage", USENIX,, 20. März 2003 (2003-03-20), Seiten 1-15, XP061009763, [gefunden am 2003-03-20]

## Beschreibung

### Gebiet der Erfindung

Die vorliegende Erfindung betrifft ein System und ein Verfahren zur sicheren serverbasierten Speicherung und Freigabe von Daten. Dabei kann ein Teilnehmer Daten auf einen Server laden und bei Bedarf die Daten für einen anderen Teilnehmer freigeben.

Bei der Speicherung sensibler Daten, beispielsweise Patienten- oder Termindaten, ist es wichtig, dass Dritte keinen Zugriff auf diese Daten erlangen können. Oftmals müssen die Daten jedoch auf einem Server bei einem externen Dienstleister gespeichert werden, um Skalierungsvorteile bei der Speicherung auszunutzen, den Administrationsaufwand zu senken oder auf die Daten von beliebigen Orten aus zugreifen zu können.

Um auf Daten weltweit Zugriff zu haben, ist es zudem oft erforderlich, mit unterschiedlichen Computersystemen zu arbeiten. Dabei kann man nicht davon ausgehen, dass ein System bestimmte Hardwarekomponenten unterstützt oder Software installiert werden kann. Der kleinste gemeinsame Nenner für einen Datenaustausch zwischen einem Clientrechner und einem Server ist oft ein Webbrowser.

### Stand der Technik

Verschlüsselte Speicherung von Daten ist gängig und weit verbreitet. Dabei werden normalerweise symmetrische Verschlüsselungsalgorithmen wie zum Beispiel AES eingesetzt. Für in das Betriebssystem integrierte Speicherung von Daten werden allerdings spezielle Software oder spezielle Treiber benötigt, deren Vorhandensein nicht überall sichergestellt werden kann.

Der sichere Austausch von Daten ist ebenfalls weit verbreitet und wird zum Beispiel im S/MIME-Verfahren zum Versenden verschlüsselter E-Mails eingesetzt. Hierbei werden asymmetrische Verschlüsselungsalgorithmen wie etwa RSA verwendet. Daten können so zwischen Parteien so ausgetauscht werden, dass Dritte nicht auf diese Daten zugreifen können.

Das SSL-Verfahren ist die verbreitetste Methode Daten, die über das Internet übertragen werden, zu verschlüsseln. Dort werden der Server und manchmal auch der Client über eine Public-Key-Infrastruktur authentifiziert, und die Daten bei der Übertragung mit standardisierten Algorithmen verschlüsselt. So können sie von Dritten nicht verändert oder abgehört werden. Hierbei wird jedoch nur der Übertragungsweg zum Server gesichert; Auf dem Server selbst liegen die Daten im Klartext vor. Dies ist für viele Sicherheitsanforderungen nicht ausreichend.

Wenn Daten auf einem Server sicher gespeichert und über den Server sicher ausgetauscht werden sollen, muss gewährleistet sein, dass die Daten nur verschlüsselt übertragen an den Server übertragen, dort verschlüsselt abgelegt und nur auf den mit dem Server verbundenen Clients entschlüsselt werden. Auf diese Weise können Dritte, die Zugriff auf die Übertragungswege oder den Server haben, die Daten nicht einsehen. Bei den im Stand der Technik bekannten Verfahren und Systemen werden die Schlüssel, mit denen die auszutauschenden Daten verschlüsselt sind, bei den Clients in lokalen, persistenten Speichern gespeichert. Dies ist nachteilig, weil beispielsweise Webbrowser oft keinen lokalen Speicher für Schlüssel besitzen, auf den man aus Browseranwendungen heraus zugreifen kann.

Eine Aufgabe der vorliegenden Erfindung ist es daher, eine sichere serverbasierte Speicherung und einen sicheren serverbasierten Austausch von Daten zu ermöglichen, ohne dass Schlüssel zur Ver- und Entschlüsselung der Daten in persistenten Speichern lokal auf einem Client gespeichert werden müssen.

US 7 010 689 A1 offenbart ein Verfahren zum Steuern eines Zugriffs auf digitale Daten in einer Datei durch Erhalten eines Passworts von einem Benutzer und Erzeugen eines persönlichen Schlüssels basierend auf dem Passwort. Ein Dateischlüssel wird erzeugt und die digitale Daten in der Datei mit dem Dateischlüssel verschlüsselt, um eine verschlüsselte Datei bereitzustellen. Der Dateischlüssel wird mit dem persönlichen Schlüssel verschlüsselt, um einen verschlüsselten Dateischlüssel bereitzustellen. Ein Datei-Header, der mit der Datei assoziiert ist und den verschlüsselten Dateischlüssel enthält, kann mit der verschlüsselten Datei auf einem Server gespeichert werden.

US 2002 076 042 offenbart ein System, welches sich auf die Erzeugung von kryptographischen Schlüsseln und deren Verwendung in kryptographischen Systemen bezieht.

US 6 694 025 B1 offenbart ein Verteilungssystem für kryptographische Schlüssel, worin die zentrale Speicherung von verschlüsselten Privatschlüsseln auf einem Server es ermöglicht, auf diese von einem Z

Client, der sowohl für den Administrator als auch für einen neuen Benutzer geeignet ist, zuzugreifen.

### Zusammenfassung der Erfindung

Diese Aufgabe wird durch die Gegenstände der unabhängigen Ansprüche gelöst. Die abhängigen Ansprüche beschreiben vorteilhafte Ausführungsformen.

Ein erfindungsgemäßes System zur sicheren serverbasierten Speicherung und Freigabe von Daten, umfasst einen Server und einen ersten Client und einen zweiten Client, die mit dem Server verbunden sind. Der erste Client ist dazu eingerichtet, einen symmetrischen Schlüssel des ersten Clients unter Verwendung von von einem Benutzer eingegebenen Daten zu erhalten. Weiterhin ist der erste Client dazu eingerichtet, zur Registrierung beim Server ein aus einem privaten Schlüssel und einem öffentlichen Schlüssel bestehendes asymmetrisches Schlüsselpaar zu erhalten, den privaten Schlüssel mit dem symmetrischen Schlüssel des ersten Clients zu verschlüsseln, den verschlüsselten privaten Schlüssel an den Server zu übertragen und den öffentlichen Schlüssel so zu veröffentlichen, dass ihn der zweite Client empfangen kann. Schließlich ist der erste Client auch dazu eingerichtet, zum Erhalten von Daten, die vom zweiten Client für den ersten Client freigegeben wurden, den verschlüsselten privaten Schlüssel vom Server zu empfangen und mit dem symmetrischen Schlüssel des ersten Clients zu entschlüsseln, und die Daten als Bereitstellungsdaten in verschlüsselter Form vom Server zu empfangen und unter Zuhilfenahme des unverschlüsselten privaten Schlüssels zu entschlüsseln. Der zweite Client ist dazu eingerichtet ist, einen symmetrischen Schlüssel des zweiten Clients unter Verwendung von von einem Benutzer eingegebenen Daten zu erhalten. Der zweite Client ist weiterhin dazu eingerichtet, zur Speicherung von Daten auf dem Server die Daten mit dem symmetrischen Schlüssel des zweiten Clients zu verschlüsseln und die verschlüsselten Daten an den Server zu übertragen. Schließlich ist der zweite Client auch dazu eingerichtet, zur Freigabe der auf dem Server gespeicherten verschlüsselten Daten für den ersten Client die verschlüsselten Daten vom Server zu empfangen und mit dem symmetrischen Schlüssel des zweiten Clients zu entschlüsseln, den öffentlichen Schlüssel des ersten Clients zu empfangen und die unverschlüsselten Daten zu verschlüssein, um Bereitstellungsdaten zu erhalten, und die Bereitstellungsdaten an den Server zu übertragen. Der Server ist dazu eingerichtet ist, die verschlüsselten Daten und den verschlüsselten privaten Schlüssel des ersten Clients zu speichern.

Das erfindungsgemäße System hat den Vorteil, dass die Daten und der private Schlüssel zu keinem Zeitpunkt unverschlüsselt auf dem Server abgelegt werden. Die Daten können derart auslesesicher auf dem Server gespeichert werden, dass nicht einmal die Administratoren Servers die Daten lesen können.

Trotzdem die Daten auf dem Server verschlüsselt abgelegt werden, müssen bei den Clients keine persistenten sensiblen Daten wie zum Beispiel symmetrische oder private Schlüssel gespeichert werden. Es ist gewährleistet, dass der private Schlüssel und die symmetrischen Schlüssel nach Beendigung der entsprechenden Browser-Sitzung an den Client-Rechnern nicht verloren gehen. Da kein persistenter Speicher benötigt wird, lassen sich die benötigten Client-Applikationen zum Beispiel als Javascript-Anwendungen implementieren, die in einem normalen Webbrowser lauffähig sind.

Die symmetrischen Schlüssel der Clients können von den Clients jederzeit unter Verwendung von von einem Benutzer eingegebenen Daten erhalten werden. Bei diesen Daten kann es sich beispielsweise um eine Phrase (Passwort) handeln, die sich der Benutzer leicht merken kann. Der Benutzer kann zum Eingeben der Phrase am Client-Rechner aufgefordert werden. Die Daten könnten aber auch z.B. auf einer Chipkarte gespeichert und vom Benutzer über einen Chipkartenleser eingeben werden. Schließlich könnten die Daten auch zum Beispiel aus dem Fingerabdruck (oder einem anderen biometrischen Merkmal) eines Benutzers reproduzierbar erzeugt und über einen Fingerabdruckscanner eingegeben werden.

Der Server und die Clients sind typischerweise Computer, auf denen ein entsprechendes Computerprogramm läuft. Die Clients können mit dem Server beispielsweise über das Internet verbunden sein. Ein Client-Rechner kann Anweisungen vom Server empfangen, die den Client-Rechner in die Lage versetzen, gemäß der Erfindung zu arbeiten. Beispielsweise wird das Clientprogramm auf dem InternetBrowser durchgeführt, der auf dem Client-Rechner läuft. Der Server stellt hierfür Anweisungen, beispielsweise in JavaScript, auf seiner Webseite bereit.

Das asymmetrische Schlüsselpaar kann auf der Grundlage des RSA-Algorithmus bestimmt werden. Für jede Applikation kann ein spezifisches asymmetrisches Schlüsselpaar erzeugt werden. Der zweite Client holt sich den geeigneten, das heißt seiner gewünschten Applikation zugeordneten, öffentlichen Schlüssel des ersten Clients, dem er seine Daten bereitstellen möchte.

Eine Möglichkeit, den öffentlichen Schlüssel so zu veröffentlichen, dass er vom zweiten Client empfangen werden kann, ist ihn auf dem Server abzulegen. Da der öffentliche Schlüssel nicht geheim ist, kann er unverschlüsselt auf dem Server abgelegt oder anderweitig veröffentlicht werden.

Der zweite Client kann beispielsweise ein Kundenrechner sein, während der erste Client ein Rechner eines Dienstleisters ist. Der Kunde kann so Daten für den Dienstleister freigeben. Beispielsweise könnte der Kunde ein Patient und der Dienstleister ein Arzt sein. Die Daten könnten Daten aus der Krankenakte des Patienten sein.

Eine Kundenkennung kann zum Zuordnen der Kundendaten an den Server übertragen werden, oder das Verfahren kann innerhalb einer vom Server bereitgestellten Sitzung ablaufen, welche mit einer Kundenkennung in Verbindung steht, um auf diese Weise eine Zuordnung der Kundendaten zur Kundenkennung bereitzustellen.

In einer Ausführungsform der Erfindung ist ein Client (2, 3) dazu eingerichtet ist, den symmetrischen Schlüssel des Clients (2, 3) dadurch zu erhalten, dass er auf der Grundlage der vom Benutzer eingegebenen Daten erzeugt wird.

Dabei kann der symmetrische Schlüssel des Clients aus den vom Benutzer eingegebenen Daten (etwa aus einer Phrase) direkt und reproduzierbar (deterministisch) erzeugt werden. Beispielsweise kann der Schlüssel ein SHA1-Hash aus einer vom Benutzer eingegebenen Phrase sein.

In einer alternativen, bevorzugten Ausführungsform der Erfindung ist ein Client dazu eingerichtet ist, den symmetrischen Schlüssel des Clients zu erzeugen und unter Verwendung der vom Benutzer eingegebenen Daten zu verschlüsseln und den verschlüsselten symmetrischen Schlüssel des Clients an den Server zu übertragen. Weiterhin ist der Client dazu eingerichtet, den verschlüsselten symmetrischen Schlüssel des Clients vom Server zu empfangen und unter Verwendung der vom Benutzer eingegebenen Daten zu entschlüsseln, um den symmetrischen Schlüssel des Clients zu erhalten. Der Server ist dazu eingerichtet, den verschlüsselten symmetrischen Schlüssel eines Clients zu speichern.

In dieser Ausführungsform wird der symmetrische Schlüssel eines Clients also dadurch erhalten, dass er vom Server heruntergeladen wird, wo er vorher verschlüsselt abgelegt wurde. Den Schlüssel zur Entschlüsselung des verschlüsselten symmetrischen Schlüssels des Clients kann der Client reproduzierbar aus den durch den Benutzer eingegebenen Daten (etwa aus einer Phrase) direkt erzeugen.

In einer weiteren Ausführungsform der Erfindung ist der symmetrische Schlüssel des zweiten Clients einer Applikation zugeordnet.

Beispielsweise ist für eine Terminbuchung ein Schlüsselpaar zu erzeugen und für ein Adressbuch ein davon abweichendes Schlüsselpaar zu erzeugen.

In einer weiteren Ausführungsform der Erfindung ist ein Client dazu eingerichtet, über einen Browser auf den Server zuzugreifen und bei einer vorhersehbar lang dauernden Ver- und/oder Entschlüsselungsoperation eine Zeitüberschreitungsbehandlung durch den Browser zu umgehen.

Da kryptografische Prozeduren unter Umständen lange Laufzeiten erfordern, ist ein derartiger Mechanismus vorgesehen. Hierzu wird zum Beispiel durch den JavaScript-Code ein Java-Applet gestartet, welches die eigentliche kryptografische Prozedur ausführt.

In einer Ausführungsform der Erfindung ist der zweite Client dazu eingerichtet, zum Erhalten der Bereitstellungsdaten die unverschlüsselten Daten mit dem öffentlichen Schlüssel des ersten Clients zu verschlüsseln. Der erste Client ist dazu eingerichtet, die vom Server empfangenen Bereitstellungsdaten mit dem unverschlüsselten privaten Schlüssel des ersten Clients zu entschlüsseln.

In einer alternativen, bevorzugten Ausführungsform der Erfindung ist der zweite Client dazu eingerichtet, zum Erhalten der Bereitstellungsdaten die unverschlüsselten Daten mit einem temporär erzeugten symmetrischen Sitzungsschlüssel zu verschlüsseln, den symmetrischen Sitzungsschlüssel mit dem öffentlichen Schlüssel des ersten Clients zu verschlüsseln und den verschlüsselten symmetrischen Sitzungsschlüssel zusammen mit den Bereitstellungsdaten an den Server zu übertragen. Der erste Client ist dazu eingerichtet, den verschlüsselten symmetrischen Sitzungsschlüssel zusammen mit den Bereitstellungsdaten vom Server zu empfangen den verschlüsselten symmetrischen Sitzungsschlüssel mit dem unverschlüsselten privaten Schlüssel des ersten Clients zu entschlüsseln und die vom Server empfangenen Bereitstellungsdaten mit dem unverschlüsselten symmetrischen Sitzungsschlüssel zu entschlüsseln.

Diese Ausführungsform erleichtert eine akzeptable Geschwindigkeit des Datenzugriffs. Hierfür ist es praktisch, wenn die Daten symmetrisch verschlüsselt gespeichert sind und mit dem symmetrischen Schlüssel entschlüsselt werden können, da symmetrische Verschlüsselungsoperationen regelmäßig schneller als asymmetrische Verschlüsselungsoperationen sind. Dies ist insbesondere wichtig, wenn die Client-Systeme nur über geringe Rechenkapazität verfügen.

In einer Ausführungsform der Erfindung weist der erste Client auch die Funktionalität des zweiten Clients auf, und der zweite Client weist auch die Funktionalität des ersten Clients auf.

Somit können beide Clients auf dem gleichen Programmcode beruhen. Beide Clients können Daten auf den Server laden und für den anderen Client freigeben. Dabei kann ein Client zur Verschlüsselung von auf dem Server zu speichernden Daten einen symmetrischen Schlüssel verwenden und bei der Verschlüsselung seines privaten Schlüssels einen anderen symmetrischen Schlüssel verwenden.

In einem erfindungsgemäßen Verfahren zur sicheren serverbasierten Speicherung und Freigabe von Daten erhält ein erster Client einen symmetrischen Schlüssel des ersten Clients unter Verwendung von von einem Benutzer eingegebenen Daten. Der erste Client erhält einen aus einem privaten Schlüssel und einem öffentlichen Schlüssel bestehendes asymmetrisches Schlüsselpaar. Der erste Client verschlüsselt den privaten Schlüssel mit dem symmetrischen Schlüssel des ersten Clients. Der verschlüsselte private Schlüssel wird vom ersten Client an einen Server übertragen. Der Server speichert den verschlüsselten privaten Schlüssel. Der öffentliche Schlüssel wird so veröffentlicht, dass ihn ein zweiter Client empfangen kann. Der zweite Client erhält einen symmetrischen Schlüssel des zweiten Clients unter Verwendung von von einem Benutzer eingegebenen Daten. Er verschlüsselt Daten mit dem symmetrischen Schlüssel des zweiten Clients. Die verschlüsselten Daten werden vom zweiten Client an den Server übertragen und auf dem Server gespeichert. Zur Freigabe der Daten werden die verschlüsselten Daten vom Server wieder an den zweiten Client übertragen. Dieser entschlüsselt die verschlüsselten Daten mit dem symmetrischen Schlüssel des zweiten Clients. Er empfängt den öffentlichen Schlüssel des ersten Clients. Er verschlüsselt die unverschlüsselten Daten, um Bereitstellungsdaten zu erhalten. Die Bereitstellungsdaten werden vom zweiten Client an den Server übertragen. Der verschlüsselte private Schlüssel wird vom Server an den ersten Client übertragen. Der erste Client entschlüsselt den verschlüsselten privaten Schlüssels mit dem symmetrischen Schlüssel des ersten Clients. Die Bereitstellungsdaten werden in verschlüsselter Form vom Server an den ersten Client überragen. Der erste Client entschlüsselt die Bereitstellungsdaten unter Zuhilfenahme des unverschlüsselten privaten Schlüssels des ersten Clients.

Die Schritte des Verfahrens müssen nicht in der Reihenfolge ausgeführt werden, in der sie oben aufgelistet wurden.

### Kurze Beschreibung der Zeichnungen

In den Zeichnungen zeigt:
- Fig. 1: eine erfindungsgemäße Internet-gestützte Anordnung zur Online-Terminbuchung von Terminen bei einem Dienstleister durch Kunden;
- Fig. 2: eine Abwandlung der Anordnung nach Fig. 1;
- Fig. 3: ein Internet-gestütztes Verfahren zur sicheren serverbasierten Speicherung und Freigabe von Kundendaten;
- Fig. 4: eine Abwandlung des Verfahrens nach Fig. 3;
- Fig. 5: ein ausführliches Beispiel einer Schlüsselerzeugung auf einem Client-Rechner;
- Fig. 6: ein Beispiel einer applikationsbezogenen Verschlüsselung;
- Fig. 7: ein Beispiel für eine gemeinsame Nutzung von Daten;

### Ausführliche Beschreibung bevorzugter Ausführungsformen

Im Folgenden werden bevorzugte Ausführungsformen der Erfindung beispielhaft beschrieben.

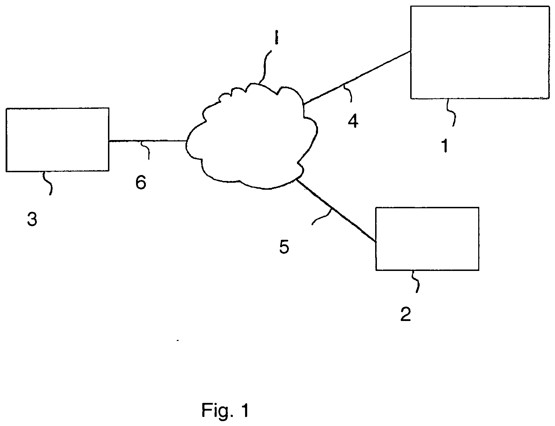

Fig. 1 zeigt eine erfindungsgemäße bevorzugte Internet-gestützte Anordnung zur Online-Terminbuchung von Terminen bei einem Dienstleister durch Kunden. Ein Server 1 ist über eine bidirektionale Verbindung 4 mit dem Internet I verbindbar, ein Dienstleister-Rechner 2 ist über eine Verbindung 5 und ein Kunden-Rechner 3 ist über eine Verbindung 6 mit dem Internet I verbindbar. Der Dienstanbieter braucht somit keine eigene Internetpräsenz zu pflegen.

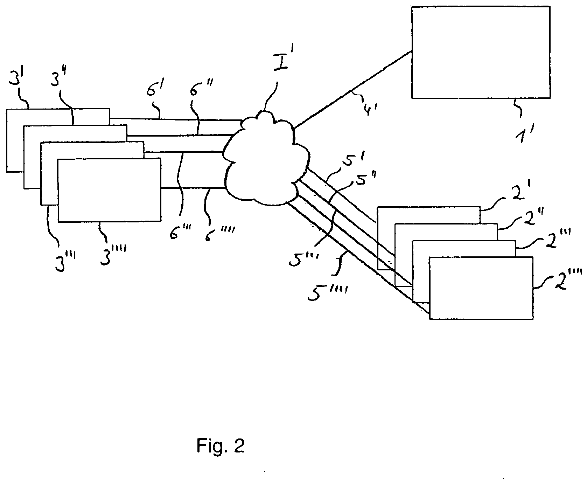

Fig. 2 zeigt eine vorteilhafte Abwandlung der Anordnung nach Fig. 1, bei der eine Mehrzahl von Dienstleister-Rechnern 2', 2", 2''' und 2'''' und eine Mehrzahl von Kunden-Rechnern 3', 3", 3''' und 3"" mit dem Internet I' verbindbar sind. Im Übrigen bedeuten gleiche Bezugszeichen die gleichen Komponenten. Eine Vielzahl von Dienstleistern teilt sich somit den einen Server 1', so dass einer Vielzahl von Kunden ein dementsprechend großes Leistungsangebot zur Verfügung steht.

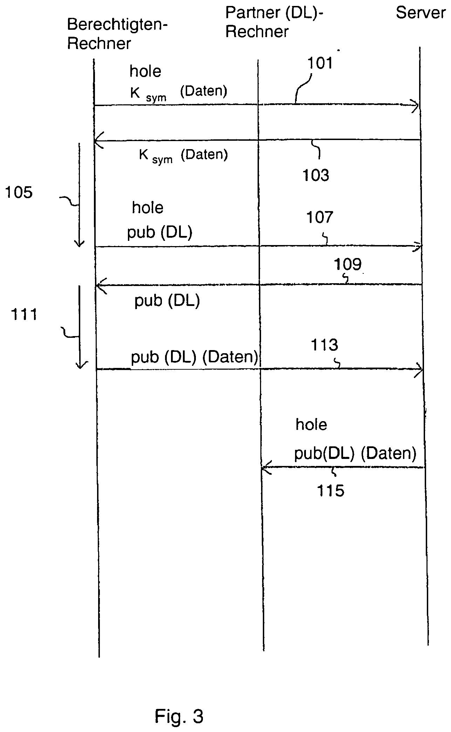

Fig. 3 zeigt ein Internet-gestütztes Verfahren zur sicheren serverbasierten Speicherung und Freigabe von Kundendaten. Kundendaten sind sicher auf einem Server gespeichert, wenn sie verschlüsselt gespeichert sind. Der Berechtigte hat vor der Verschlüsselung und serverbasierten Speicherung der Daten auf Grundlage einer nur ihm bekannten Phrase einen symmetrischen Schlüssel Ksym in an sich bekannter Weise erzeugt. Mit dem Schlüssel Ksym verschlüsselt er seine Daten und überträgt sie zur Speicherung an den Server. Ferner erzeugt der Berechtigte auf seinem Client-Rechner ein asymmetrisches Schlüsselpaar, beispielsweise mit dem RSA-Algorithmus. Um einen sicheren Datenaustausch zu Verfügung zu stellen, überträgt der Berechtigte seinen öffentlichen Schlüssel unverschlüsselt an den Server zur Speicherung. Ferner verschlüsselt er den privaten Schlüssel mit dem symmetrischen Schlüssel und hinterlegt den so verschlüsselten privaten Schlüssel auf dem Server. Der Berechtigte kann dann problemlos den Browser beenden, weil alle Schlüssel sicher auf dem Server gespeichert sind.

In einer bevorzugten erfindungsgemäßen Ausführungsform, wie in Fig. 3 gezeigt, sendet der Berechtigte vom Berechtigten-Rechner zunächst eine Anforderung 101 an den Server, die mit dem symmetrischen Schlüssel Ksym verschlüsselten Daten Ksym(Daten) zu holen. Der Server überträgt dem Berechtigten die Daten Ksym(Daten). In einem Schritt 105 entschlüsselt der Berechtigten-Rechner auf der Grundlage der vom Server bereitgestellten Software in einem Browser die verschlüsselten Daten Ksym(Daten) mit dem symmetrischen Schlüssel Ksym. In einem Schritt 107 fordert der Berechtigte einen öffentlichen Schlüssel pub(DL) des Partners an, dem er die Kundendaten bereitstellen möchte. In einem Schritt 111 verschlüsselt der Berechtigte die Kundendaten mit dem öffentlichen Schlüssel pub(DL) des Partner und erhält so die verschlüsselten Daten pub(DL)(Daten), die als Bereitstellungsdaten dienen. Der Berechtigte überträgt dann die Bereitstellungsdaten in einem Schritt 113 an den Server. Der Partner holt sich schließlich die Daten in einem Schritt 115 auf seinen Partner-Rechner und entschlüsselt die Daten pub(DL)(Daten) mit dem zugehörigen privaten Schlüssel in an sich bekannter Weise.

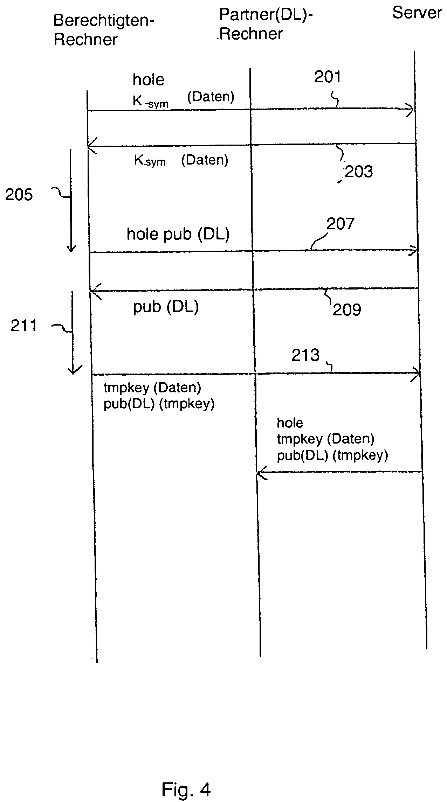

Fig. 4 zeigt eine andere bevorzugte Ausführungsform des Intemet-gestützten Verfahrens zur sicheren serverbasierten Speicherung und Freigabe von Kundendaten nach Fig. 3. Ähnliche Bezugszeichen bedeuten dabei ähnliche Schritte.

Der Unterschied zum vorangegangenen Verfahren ist, dass im Schritt 211 ein temporärer symmetrischer Schlüssel tmpkey erzeugt wird. Eine Phrase ist für die Erzeugung des temporären symmetrischen Schlüssels nicht zwingend erforderlich. Die Kundendaten werden dann mittels des temporären symmetrischen Schlüssels tmpkey verschlüsselt. Der temporäre symmetrische Schlüssel tmpkey wird mit dem öffentlichen Schlüssel des Partners pub(DL) verschlüsselt. Auf diese Weise wird eine bei größeren Datenmengen zeitintensive Verschlüsselung mit einem öffentlichen Schlüssel abgekürzt, indem nur der temporäre symmetrische Schlüssel tmpkey verschlüsselt wird, der nur einen geringen Datenumfang einnimmt. Danach werden die mit dem temporären symmetrischen Schlüssel tmpkey verschlüsselten Daten als Bereitstellungsdaten tmpkey(Daten) zusammen mit dem verschlüsselten temporären symmetrischen Schlüssel pub(DL)(tmpkey) übertragen. Der Partner holt sich dann die Bereitstellungsdaten tmpkey(Daten) und den verschlüsselten temporären symmetrischen Schlüssel pub(DL)(tmpkey) vom Server, entschlüsselt den verschlüsselten Schlüssel mit seinem privaten Schlüssel und entschlüsselt schließlich mit dem so wiedergewonnenen temporären symmetrischen Schlüssel in an sich bekannter Weise die Kundendaten.

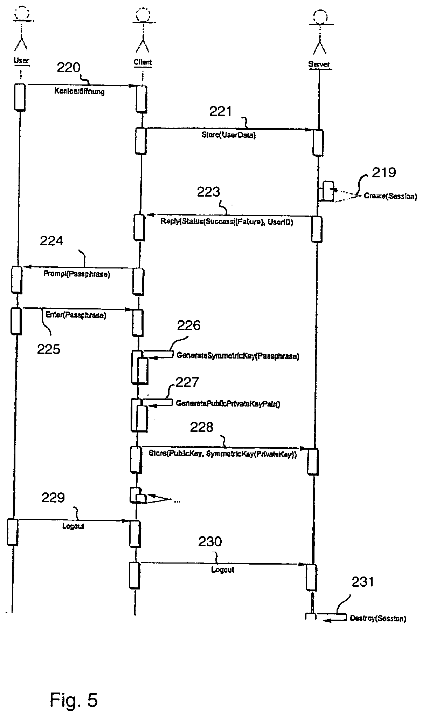

Fig. 5 zeigt ein ausführliches Beispiel einer Schlüsselerzeugung auf einem Client-Rechner. Ein Nutzer (User), der sich auf dem erfindungsgemäß eingerichteten Server registrieren möchte, fordert über seinen Client, der ein Internetbrowser sein kann, eine Kontoeröffnung an, 220. Der Client sendet eine Anforderung zum Speichern der Nutzerdaten an den Server, 221, der in einem Schritt 219 daraufhin eine Sitzung anlegt (Create (Session)) und dem Client eine Statusmeldung über Erfolg oder Misserfolg sowie eine Nutzerkennung UserID mitteilt, 223. Daraufhin fordert der Client den Nutzer auf, eine Phrase anzugeben, 224. Sobald der Nutzer eine Phrase angegeben hat, 225, erzeugt der Client lokal einen symmetrischen Schlüssel unter Verwendung der Phrase, 226, und ein aus einem privaten Schlüssel und einem öffentlichen Schlüssel bestehendes Schlüsselpaar, 227. Den öffentlichen Schlüssel und den mit dem symmetrischen Schlüssel verschlüsselten privaten Schlüssel überträgt der Client zur Speicherung an den Server, 228, da nach Beendigung des Clients, d.h. des Browsers, sämtliche Daten verloren sind. Nunmehr kann der Nutzer sich beim Server abmelden, 229. Der Client reicht die Abmeldung an den Server weiter, 230, der daraufhin die Sitzung beendet und den temporären Sitzungsspeicher freigibt, 231, während die Schlüssel persistent auf dem Server gespeichert werden.

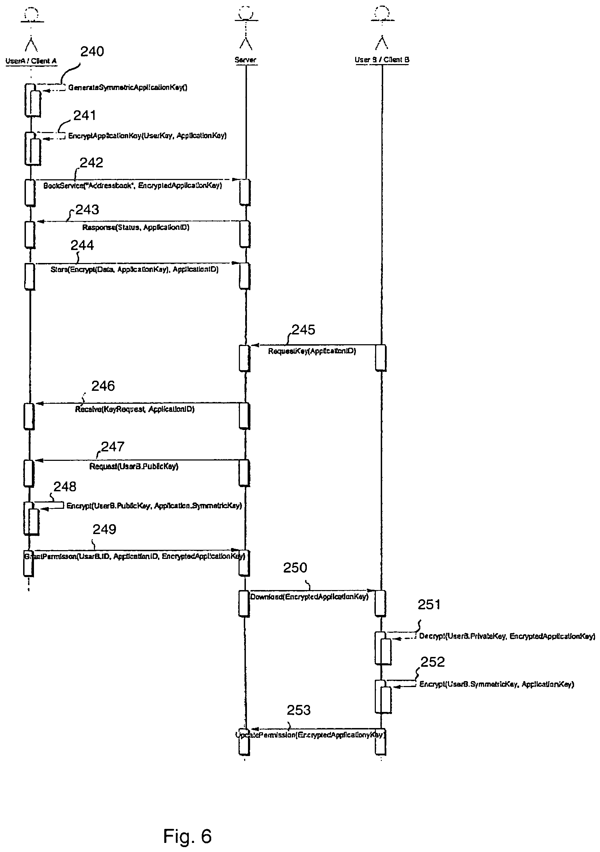

Fig. 6 zeigt ein Beispiel einer applikationsbezogenen Verschlüsselung. Eine Applikation ist beispielsweise ein Adressbuch, das mit eigenen zugeordneten Schlüsseln zu betreiben ist. In 240 erzeugt der Nutzer A auf dem Client A einen symmetrischen Applikationsschlüssel. Der Applikationsschlüssel wird in 241 mit dem Nutzerschlüssel verschlüsselt. Hernach wird die Applikation "Adressbuch" unter Angabe des verschlüsselten Applikationsschlüssels beim Server gebucht, 242. Der Server antwortet mit einer Statusmeldung und einer Applikationskennung, 243, woraufhin es dem Client A möglich ist, unter der mitgeteilten Applikationskennung mit dem Applikationsschlüssel verschlüsselte Adressdaten auf dem Server zu speichern, 244. Fordert nun ein Client B einen Schlüssel für die der Applikationskennung zugeordnete Applikation an, 245, so reicht der Server diese Anforderung an den Client A, 246, der daraufhin zur Aufnahme eines Austauschs mit dem Client B den öffentlichen Schlüssel vom Client B anfordert, 247. Mit dem erhaltenen öffentlichen Schlüssel vom Client B verschlüsselt der Client A daraufhin seinen symmetrischen Applikationsschlüssel. In 249 gewährt der Client A dem Client B den Zugriff auf seine, also des Clients A, Applikationsdaten. Der Client B lädt sich vom Server den verschlüsselten Applikationsschlüssel herunter, 250, und entschlüsselt den Applikationsschlüssel mit seinem privaten Schlüssel, 251. In 252 verschlüsselt der Client B mit seinem symmetrischen Schlüssel den erhaltenen Applikationsschlüssel und speichert ihn auf dem Server, 253, um die Zugriffsgewährung zu aktualisieren.

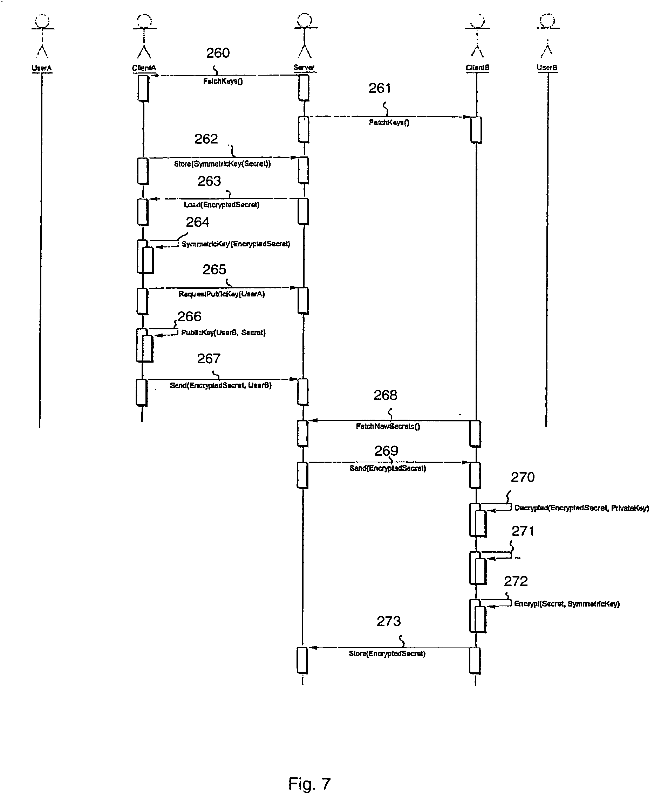

Fig. 7 ein Beispiel für eine gemeinsame Nutzung von Daten. In nicht abgebildeten Schritten wird in diesem Ausführungsbeispiel auf den Clients beim Registrieren jeweils der symmetrische Schlüssel des Clients erzeugt. Dieser wird dann mit einem weiteren symmetrischen Schlüssel verschlüsselt und so auf dem Server abgelegt. Der angesprochene weitere symmetrische Schlüssel wird aus vom Benutzer eingegebenen Daten wie einer Phrase reproduzierbar erzeugt. In Schritten 260 und 261 holen sich ein Client A und ein Client B ihre jeweiligen verschlüsselten symmetrischen Schlüssel vom Server. In nicht dargestellten Schritten geben die jeweiligen Benutzer Daten (z.B. eine Phrase) ein, aus denen die weiteren symmetrischen Schlüssel zur Entschlüsselung der vom Server geholten symmetrischen Schlüssel erneut erzeugt werden. Der Client A speichert daraufhin mit dem entschlüsselten symmetrischen Schlüssel verschlüsselte Kundendaten auf dem Server, 262. In einem folgenden Schritt 263 holt sich der Client A seine mit einem symmetrischen Schlüssel verschlüsselte Kundendaten und entschlüsselt sie in einem Schritt 264. Um einen Datenaustausch mit dem Client B aufnehmen zu können, fordert Client A den öffentlichen Schlüssel des Clients B vom Server an, 265, mit dem er seine Kundendaten verschlüsselt, 266. Hernach überträgt der Client A die mit dem öffentlichen Schlüssel von dem Client B verschlüsselten Daten an den Server. Der Client B startet in 268 eine Anfrage nach neuen Kundendaten an den Server, die dieser dem Client B in 269 zusendet. Im Schritt 270 entschlüsselt der Client B die erhaltenen Kundendaten mit seinem privaten Schlüssel und nimmt in 271 eine Aktualisierung der Kundendaten vor. Hernach verschlüsselt der Client B die Kundendaten mit seinem symmetrischen Schlüssel in 272 und speichert die verschlüsselten Kundendaten auf dem Server.

Die Erfindung kann verwendet werden für eine Verbindung zwischen der Gesundheitsakte eines Patienten und eines Online-Praxismanagements.

Ein besonderer Vorteil einer Ausführungsform der Erfindung liegt darin, dass die Verschlüsselung in einer Web-Anwendung (in JavaScript) abläuft und einen Freigabemechanismus enthält, der es dem Nutzer ermöglicht Dritten (in unserem Fall Ärzte) die verschlüsselt gespeicherten Daten komplett oder auch nur in Teilen zur Verfügung zu stellen, während die Nutzdaten während der Übertragung durch das Internet als auch auf dem Server selbst nur in verschlüsselter Form vorliegen.

Dies ergänzt Verfahren wie Verschlüsselung der Kommunikation mittels SSL oder Datenträgerverschlüsselung auf dem Server. SSL sichert die Kommunikation von Parteien über ein unsicheres Netzwerk, indem die übertragenen Daten asymmetrisch verschlüsselt und signiert werden. Auf dem Server werden sie jedoch wieder entschlüsselt und sind dort potentiell von Serveradministratoren lesbar. Verschlüsselte Datenträger stellen sicher, dass die Daten auf dem Server bei Verlust oder Diebstahl des Datenträgers nicht entschlüsselt werden können (da der zur Entschlüsselung nötige Schlüssel in flüchtigem Speicher auf dem Server gespeichert ist), sind jedoch auch hier während der Server aktiv ist vor Serveradministratoren nicht geschützt.

Die Daten werden direkt im Browser verschlüsselt, d.h. die Nutzer können damit völlig ohne Chipkarten-Lesengeräte auskommen.

Die oben ausführlich dargestellten kryptografischen Schritte sind wie folgt stichpunktartig zusammengefasst:
Benutzer (alle Vorgänge laufen auf dem Client ab)
- JS RSA-Schlüsselerzeugung (ergibt einen öffentlichen Schlüssel pub und einen privaten Schlüssel priv)
- JS Passwort2Key (ergibt symmetrischen Schlüssel ksym)
- JS AES-Verschlüsselung (des privaten Schlüssels mit dem symmetrischen Schlüssel, ergibt ksym(priv))
- Speicherung des verschlüsselten priv auf dem Server
- Mit dem Nutzer wird auf dem Client ein zufälliger Benutzerschlüssel U erzeugt, mit einem Passwort (einer Phrase) P verschlüsselt und auf dem Server gespeichert.
- Für jede Ver-/Entschlüsselung ist zunächst der Benutzerschlüssel herunterzuladen und zu entschlüsseln

Passwort-Änderung:
- Benutzerschlüssel herunterladen, entschlüsseln, neu verschlüsseln, wieder auf Server speichern

Datenschlüssel (Fallschlüssel) je Fall/Datum:
- Für jeden Datensatz wird zufälliger Fallschlüssel F generiert, womit die Felder verschlüsselt werden. Dieser wird mit U verschlüsselt (der dafür heruntergeladen und mit P geöffnet wird).

Anlage und Abruf von Datensätzen:
- Verschlüsselung auf dem Client mit zufälligem Datenkey
- Verschlüsselung des Datenkeys mit Passwort2Key
- Speicherung von verschlüsseltem key und Nutzdaten auf Server

Freigabe:
- Abruf der Daten wie unter "Anlage und Abruf von Datensätzen"
- Verschlüsselung mit zufälligem symmetrischen Schlüssel key
- Herunterladen des öffentlichen Schlüssels pubkey des Arztes
- Verschlüsselung des zufälligen key mit pubkey des Arztes
- Hochladen der Nutzdaten und des für den Arzt verschlüsselten key

Annahme der Freigabe auf Arztseite ("Update-Queue")
- Herunterladen neuer Nutzdaten, Schlüssel dazu und verschlüsselten eigenen privaten Schlüssel durch Arzt
- Entschlüsselung des eigenen privaten Schlüssels mit Passwort2Key
- Entschlüsselung des Nutzdatenschlüssel
- Entschlüsselung der Nutzdaten
- weiter wie bei Anlage und Abruf von Datensätzen

Die vorliegende Erfindung wurde beispielhaft im Rahmen einer Patient-Arzt-Beziehung beschrieben, ist aber grundsätzlich auf alle Kunden-Dienstleister-Beziehungen anwendbar.

Insbesondere schafft die vorliegende Erfindung System zur sicheren serverbasierten Speicherung und Freigabe von Daten sowie ein dieses System betreibendes Verfahren.

## Patentansprüche

1. System zur sicheren serverbasierten Speicherung und Freigabe von Daten, umfassend einen Server (1); und
einen ersten Client (2) und einen zweiten Client (3), die mit dem Server verbunden sind;
wobei der erste Client (2) dazu eingerichtet ist,
- einen ersten symmetrischen Schlüssel (ksym) unter Verwendung von von einem ersten Benutzer eingegebenen Daten zu erhalten;
- zur Registrierung des ersten Benutzers beim Server (1) ein aus einem ersten privaten Schlüssel und einem ersten öffentlichen Schlüssel (pub(DL)) bestehendes erstes asymmetrische Schlüsselpaar zu erhalten, den ersten privaten Schlüssel mit dem ersten symmetrischen Schlüssel (ksym) zu verschlüsseln, den verschlüsselten ersten privaten Schlüssel an den Server (1) zu übertragen und den ersten öffentlichen Schlüssel (pub(DL)) so zu veröffentlichen, dass ihn der zweite Client empfangen kann;
- zum Erhalten von Daten, die von einem zweiten Benutzer für den ersten Benutzer freigegeben wurden, den verschlüsselten ersten privaten Schlüssel vom Server (1) zu empfangen und mit dem ersten symmetrischen Schlüssel zu entschlüsseln, und die Daten als Bereitstellungsdaten in verschlüsselter Form vom Server (1) zu empfangen und unter Zuhilfenahme des unverschlüsselten ersten privaten Schlüssels zu entschlüsseln;
wobei der zweite Client (3) dazu eingerichtet ist,
- einen zweiten symmetrischen Schlüssel (ksym) unter Verwendung von von dem zweiten Benutzer eingegebenen Daten zu erhalten;
- zur Speicherung von Daten auf dem Server (1) die Daten mit dem zweiten symmetrischen Schlüssel (ksym) zu verschlüsseln und die verschlüsselten Daten an den Server (1) zu übertragen;
- zur Freigabe der auf dem Server (1) gespeicherten verschlüsselten Daten für den ersten Benutzer die Daten vom Server (1) zu empfangen, den ersten öffentlichen Schlüssel (pub(DL)) zu empfangen, die verschlüsselten Daten mit dem zweiten symmetrischen Schlüssel (ksym) zu entschlüsseln, die unverschlüsselten Daten unter Zuhilfenahme des ersten öffentlichen Schlüssels (pub(DL)) zu verschlüsseln, um Bereitstellungsdaten zu erhalten, und die Bereitstellungsdaten an den Server (1) zu übertragen; und
wobei der Server weiterhin dazu eingerichtet ist,
- die verschlüsselten Daten und
- den verschlüsselten ersten privaten Schlüssel zu speichern.

2. System nach Anspruch 1, wobei ein Client (2, 3) dazu eingerichtet ist, den symmetrischen Schlüssel (ksym) dadurch zu erhalten, dass er auf der Grundlage der vom Benutzer eingegebenen Daten erzeugt wird.

3. System nach Anspruch 1,
wobei ein Client (2, 3) dazu eingerichtet ist,
- den symmetrischen Schlüssel (ksym) zu erzeugen und unter Verwendung der vom Benutzer eingegebenen Daten zu verschlüsseln und den verschlüsselten symmetrischen Schlüssel (ksym) an den Server (1) zu übertragen;
- den verschlüsselten symmetrischen Schlüssel (ksym) vom Server (1) zu empfangen und unter Verwendung der vom Benutzer eingegebenen Daten zu entschlüsseln, um den symmetrischen Schlüssel (ksym) zu erhalten; und
wobei der Server (1) dazu eingerichtet ist, den verschlüsselten symmetrischen Schlüssel (ksym) zu speichern.

4. System nach einem der vorangegangenen Ansprüche, wobei ein Client (2, 3) dazu eingerichtet ist, über einen Browser auf den Server zuzugreifen und bei einer vorhersehbar lang dauernden Ver- und/oder Entschlüsselungsoperation eine Zeitüberschreitungsbehandlung durch den Browser zu umgehen.

5. System nach einem der vorangegangenen Ansprüche,
wobei der zweite Client (3) dazu eingerichtet ist, zum Erhalten der Bereitstellungsdaten die unverschlüsselten Daten mit dem ersten öffentlichen Schlüssel (pub(DL)) zu verschlüsseln; und
wobei der erste Client (2) dazu eingerichtet ist, die vom Server (1) empfangenen Bereitstellungsdaten mit dem unverschlüsselten ersten privaten Schlüssel zu entschlüsseln.

6. System nach einem der Ansprüche 1 bis 4,
wobei der zweite Client (3) dazu eingerichtet ist,
- zum Erhalten der Bereitstellungsdaten die unverschlüsselten Daten mit einem temporär erzeugten symmetrischen Sitzungsschlüssel (tmpkey) zu verschlüsseln;
- den symmetrischen Sitzungsschlüssel (tmpkey) mit dem ersten öffentlichen Schlüssel (pub(DL)) zu verschlüsseln; und
- den verschlüsselten symmetrischen Sitzungsschlüssel (pub(DL)(tmpkey)) zusammen mit den Bereitstellungsdaten (tmpkey(Daten)) an den Server (1) zu übertragen; und
wobei der erste Client (2) dazu eingerichtet ist,
- den verschlüsselten symmetrischen Sitzungsschlüssel (pub(DL)(tmpkey)) zusammen mit den Bereitstellungsdaten (tmpkey(Daten)) vom Server (1) zu empfangen;
- den verschlüsselten symmetrischen Sitzungsschlüssel (pub(DL)(tmpkey)) mit dem unverschlüsselten ersten privaten Schlüssel zu entschlüsseln; und
- die vom Server (1) empfangenen Bereitstellungsdaten (tmpkey(Daten)) mit dem unverschlüsselten symmetrischen Sitzungsschlüssel (tmpkey) zu entschlüsseln.

7. System nach einem der vorangegangenen Ansprüche, wobei der erste Client auch die Funktionalität des zweiten Clients aufweist, und der zweite Client auch die Funktionalität des ersten Clients aufweist.

8. Client, der dazu eingerichtet ist, als erster Client in einem System nach einem der Ansprüche 1 bis 7 eingesetzt zu werden.

9. Verfahren zur sicheren serverbasierten Speicherung und Freigabe von Daten, umfassend die Schritte:
- Erhalten eines ersten symmetrischen Schlüssels (ksym) durch den ersten Client (2) unter Verwendung von von einem ersten Benutzer eingegebenen Daten;
- Erhalten eines aus einem ersten privaten Schlüssel und einem ersten öffentlichen Schlüssel (pub(DL)) bestehenden ersten asymmetrischen Schlüsselpaars durch den ersten Client (2);
- Verschlüsseln des ersten privaten Schlüssels mit dem ersten symmetrischen Schlüssel (ksym) durch den ersten Client (2);
- Übertragen des verschlüsselten ersten privaten Schlüssels vom ersten Client (2) an einen Server (1);
- Speichern des verschlüsselten ersten privaten Schlüssels auf dem Server (1);
- Veröffentlichen des ersten öffentlichen Schlüssels (pub(DL)) so, dass ihn ein zweiter Client empfangen kann;
- Erhalten eines zweiten symmetrischen Schlüssels (ksym) durch den zweiten Client (3) unter Verwendung von von einem zweiten Benutzer eingegebenen Daten;
- Verschlüsseln von Daten mit dem zweiten symmetrischen Schlüssel (ksym) durch den zweiten Client (3);
- Übertragen der verschlüsselten Daten vom zweiten Client an den Server (1);
- Speichern der verschlüsselten Daten auf dem Server (1);
- Übertragen der verschlüsselten Daten vom Server (1) an den zweiten Client (3);
- Entschlüsseln der verschlüsselten Daten mit dem zweiten symmetrischen Schlüssel (ksym) durch den zweiten Client (3);
- Empfangen des ersten öffentlichen Schlüssels (pub(DL)) durch den zweiten Client (3);
- Verschlüsseln der unverschlüsselten Daten durch den zweiten Client (3), unter Zuhilfenahme des ersten öffentlichen Schlüssels (pub(DL)) um Bereitstellungsdaten zu erhalten;
- Übertragen der Bereitstellungsdaten vom zweiten Client (3) an den Server (1);
- Übertragen des verschlüsselten ersten privaten Schlüssels vom Server (1) an den ersten Client (2);
- Entschlüsseln des verschlüsselten ersten privaten Schlüssels mit dem ersten symmetrischen Schlüssel durch den ersten Client (2);
- Übertragen der Bereitstellungsdaten in verschlüsselter Form vom Server (1) an den ersten Client (2);
- Entschlüsseln der Bereitstellungsdaten durch den ersten Client (2) unter Zuhilfenahme des unverschlüsselten ersten privaten Schlüssels.

10. Computerprogramm, das, wenn es auf einem oder mehreren Computern ausgeführt wird bewirkt, dass das Verfahren nach Anspruch 9 durchgeführt wird.

## Claims

1. A system for secure server-based storage and sharing of data, comprising a server (1); and
a first client (2) and a second client (3) connected to the server;
wherein the first client (2) is configured to,
- obtain a first symmetric key (ksym) using data entered by a first user;
- obtain a first asymmetric key pair comprising a first private key and a first public key (pub(DL)) for registering the first user with the server (1), encrypt the first private key with the first symmetric key (ksym), transmit the encrypted first private key to the server (1), and publish the first public key (pub(DL)) so that the second client can receive it;
- obtain data shared by a second user with the first user, receive the encrypted first private key from the server (1) and decrypt it with the first symmetric key, and receive the data as provision data in encrypted form from the server (1) and decrypt it by using the unencrypted first private key;
wherein the second client (3) is configured to,
- obtain a second symmetric key (ksym) using data entered by the second user;
- encrypt data with the second symmetric key (ksym) to store the data on the server (1), and transmit the encrypted data to the server (1);
- receive the encrypted data stored on the server (1) to share with the first user, receive the first public key (pub(DL)), decrypt the encrypted data with the second symmetric key (ksym), encrypt the unencrypted data using the first public key (pub(DL)) to obtain provision data, and transmit the provision data to the server (1);
wherein the server is further configured to
- store the encrypted data and
- the encrypted first private key.

2. The system according to claim 1, wherein a client (2, 3) is configured to obtain the symmetric key (ksym) by generating it on the basis of the data entered by the user.

3. The system according to claim 1,
wherein a client (2, 3) is configured to
- generate the symmetric key (ksym) and encrypt it using the data entered by the user and transmit the encrypted symmetric key (ksym) to the server (1);
- receive the encrypted symmetric key (ksym) from the server (1) and decrypt it using the data entered by the user to obtain the symmetric key (ksym); and
wherein the server (1) is configured to store the encrypted symmetric key (ksym).

4. The system according to one of the preceding claims, wherein a client (2, 3) is configured to access the server via a browser and to bypass a time-out handling by the browser in case of a predictably long lasting encryption and/or decryption operation.

5. The system according to one of the preceding claims,
wherein the second client (3) is configured to encrypt the unencrypted data with the first public key (pub(DL)) to obtain the provision data; and
wherein the first client (2) is configured to decrypt the provision data received from the server (1) with the unencrypted first private key.

6. The system according to one of claims 1 to 4,
wherein the second client (3) is configured to
- encrypt the unencrypted data with a temporarily generated symmetric session key (tmpkey) to obtain the provision data;
- encrypt the symmetric session key (tmpkey) with the first public key (pub(DL)); and
- transmit the encrypted symmetric session key (pub(DL)(tmpkey)) together with the provision data (tmpkey(Daten)) to the server (1); and
wherein the first client (2) is configured to
- receive the encrypted symmetric session key (pub(DL)(tmpkey)) together with the provision data (tmpkey(Daten)) from the server (1)
- decrypt the encrypted symmetric session key (pub(DL)(tmpkey)) with the unencrypted first private key; and
- decrypt the provision data (tmpkey(Daten)) received from the server (1) with the unencrypted symmetric session key (tmpkey).

7. The system according to one of the preceding claims, wherein the first client also has the functionality of the second client, and the second client also has the functionality of the first client.

8. A client configured to be the first client in a system according to one of claims 1 through 7.

9. A method for secure server-based storage and sharing of data, comprising the steps of:
- obtaining a first symmetric key (ksym) by the first client (2) using data entered by a first user;
- obtaining a first asymmetric key pair comprising a first private key and a first public key (pub(DL)) by the first client (2);
- encrypting the first private key with the first symmetric key (ksym) by the first client (2);
- transmitting the encrypted first private key from the first client (2) to a server (1);
- storing the encrypted first private key on the server (1);
- publishing the first public key (pub(DL)) so that a second client can receive it;
- obtaining a second symmetric key (ksym) by the second client (3) using data entered by a second user;
- encrypting data with the second symmetric key (ksym) by the second client (3);
- transmitting the encrypted data from the second client to the server (1);
- storing the encrypted data on the server (1);
- transmitting the encrypted data from the server (1) to the second client (3);
- decrypting the encrypted data with the second symmetric key (ksym) by the second client (3);
- receiving the first public key (pub(DL)) by the second client (3);
- encrypting the unencrypted data by the second client (3), using the first public key (pub(DL)) to obtain provision data;
- transmitting the provision data from the second client (3) to the server (1);
- transmitting the encrypted first private key from the server (1) to the first client (2);
- decrypting the encrypted first private key with the first symmetric key by the first client (2);
- transmitting the provision data in encrypted form from the server (1) to the first client (2);
- decrypting the provision data by the first client (2) using the unencrypted first private key.

10. A computer program which, when executed on one or more computers, causes the method of claim 9 to be executed.

## Revendications

1. Système de stockage sûr basé sur un serveur et de libération de données, incluant un serveur (1) ; et
un premier client (2) et un deuxième client (3) qui sont connectés au capteur ; le premier client (2) étant agencé pour
- obtenir une première clé symétrique (ksym) en utilisant des données entrées par un premier utilisateur ;
- pour enregistrer le premier utilisateur sur le serveur (1), obtenir une première paire de clés asymétrique constituée d'une première clé privée et d'une première clé publique (pub(DL)), crypter la première clé privée avec la première clé symétrique (ksym), transmettre la première clé privée cryptée au serveur (1) et publier la première clé publique (pub(DL)) de façon que le deuxième client puisse la recevoir ;
- pour obtenir des données qui ont été libérées par un deuxième utilisateur pour le premier utilisateur, recevoir du serveur (1) la première clé privée cryptée et la décrypter avec la première clé symétrique, et recevoir du serveur (1) les données en tant que données de mise à disposition sous forme cryptée et les décrypter à l'aide de la première clé privée non cryptée ;
le deuxième client (3) étant agencé pour :
- obtenir une deuxième clé symétrique (ksym) en utilisant des données entrées par le deuxième utilisateur ;
- pour stocker des données sur le serveur (1), crypter les données avec la deuxième clé symétrique (ksym) et transmettre les données cryptées au serveur (1) ;
- pour libérer les données cryptées stockées sur le serveur (1) pour le premier utilisateur, recevoir les données du serveur (1), recevoir la première clé publique (pub(DL)), décrypter les données cryptées avec la deuxième clé symétrique (ksym), crypter les données non cryptées à l'aide de la première clé publique (pub(DL)) afin d'obtenir des données de mise à disposition, et transmettre les données de mise à disposition au serveur (1) ; et
le serveur étant également agencé pour
- stocker les données cryptées et
- la première clé privée cryptée.

2. Système selon la revendication 1, un client (2, 3) étant agencé pour obtenir la clé symétrique (ksym) par le fait qu'elle est générées sur la base des données entrées par l'utilisateur.

3. Système selon la revendication 1,
un client (2, 3) étant agencé pour
- générer la clé symétrique (ksym) et la crypter en utilisant les données entrées par l'utilisateur et transmettre la clé symétrique cryptée (ksym) au serveur (1) ;
- recevoir la clé symétrique cryptée (ksym) du serveur (1) et la décrypter en utilisant les données entrées par l'utilisateur afin d'obtenir la clé symétrique (ksym) ; et
le serveur (1) étant agencé pour stocker la clé symétrique cryptée (ksym).

4. Système selon une des revendications précédentes, un client (2, 3) étant agencé pour accéder au serveur par l'intermédiaire d'un navigateur et, dans le cas d'une opération de cryptage et/ou décryptage prévue pour durer longtemps, contourner un traitement de dépassement de temps par l'intermédiaire du navigateur.

5. Système selon une des revendications précédentes, le deuxième client (3) étant agencé pour crypter les données non cryptées avec la première clé publique (pub(DL)) afin d'obtenir les données de mise à disposition ; et
le premier client (2) étant agencé pour décrypter les données de mise à disposition reçues du serveur (1) avec la première clé privée non cryptée.

6. Système selon une des revendications 1 à 4,
le deuxième client (3) étant agencé pour
- pour obtenir les données de mise à disposition, crypter les données non cryptées avec une clé de session symétrique générée temporairement (tmpkey) ;
- crypter la clé de session symétrique (tmpkey) avec la première clé publique (pub(DL)) ; et
- transmettre au serveur (1) la clé de session symétrique cryptée (pub(DL)(tmpkey)) conjointement avec les données de mise à disposition (tmpkey(Daten)) ; et
le premier client (2) étant agencé pour
- recevoir du serveur (1) la clé de session symétrique cryptée (pub(DL)(tmpkey)) conjointement avec les données de mise à disposition (tmpkey(Daten)) ;
- décrypter la clé de session symétrique cryptée (pub(DL)(tmpkey)) avec la première clé privée non cryptée ; et
- décrypter les données de mise à disposition (tmpkey(Daten)) reçues du serveur (1) avec la clé de session symétrique non cryptée (tmpkey).

7. Système selon une des revendications précédentes, le premier client comportant aussi la fonctionnalité du deuxième client, et le deuxième client comportant aussi la fonctionnalité du premier client.

8. Client qui est agencé pour être mis en œuvre en tant que premier client dans un système selon une des revendications 1 à 7.

9. Procédé de stockage sûr basé sur un serveur et de libération de données, incluant les étapes de :
- obtention d'une première clé symétrique (ksym) par le premier client (2) en utilisant des données entrées par un premier utilisateur ;
- obtention, par le premier client (2), d'une première paire de clés asymétrique constituée d'une première clé privée et d'une première clé publique (pub(DL)) ;
- cryptage, par le premier client (2), de la première clé privée avec la première clé symétrique (ksym) ;
- transmission de la première clé privée cryptée du premier client (2) à un serveur (1) ;
- stockage de la première clé privée cryptée sur le serveur (1) ;
- publication de la première clé publique (pub(DL)) de façon qu'un deuxième client puisse la recevoir ;
- obtention de la deuxième clé symétrique (ksym) par le deuxième client (3) en utilisant des données entrées par un deuxième utilisateur ;
- cryptage de données avec la deuxième clé symétrique (ksym) par le deuxième client (3) ;
- transmission des données cryptées du deuxième client au serveur (1) ;
- stockage des données cryptées sur le serveur (1) ;
- transmission des données cryptées du serveur (1) au deuxième client (3) ;
- décryptage, par le deuxième client (3), des données cryptées avec la deuxième clé symétrique (ksym) ;
- réception de la première clé publique (pub(DL)) par le deuxième client (3) ;
- cryptage des données non cryptées par le deuxième client (3) à l'aide de la première clé publique (pub(DL)) pour obtenir des données de mise à disposition ;
- transmission des données de mise à disposition du deuxième client (3) au serveur (1) ;
- transmission de la première clé privée cryptée du serveur (1) au premier client (2) ;
- décryptage, par le premier client (2), de la première clé privée cryptée avec la première clé symétrique ;
- transmission des données de mise à disposition sous forme cryptée du serveur (1) au premier client (2) ;
- décryptage des données de mise à disposition par le premier client (2) à l'aide de la première clé privée non cryptée.

10. Programme informatique qui, lorsqu'il est exécuté sur un ou plusieurs ordinateurs, permet au procédé selon la revendication 9 d'être mis en œuvre.
